Europäisches Patentamt

(19) European Patent Office

Office européen des brevets

(11) **EP 0 627 898 B1**

(12) **EUROPEAN PATENT SPECIFICATION**

(45) Date of publication and mention
of the grant of the patent:
**12.11.1997 Bulletin 1997/46**

(21) Application number: **93904245.3**

(22) Date of filing: **24.02.1993**

(51) Int. Cl.$^6$: **A61B 19/00**, A61B 8/08

(86) International application number:
**PCT/GB93/00374**

(87) International publication number:
**WO 93/16652 (02.09.1993 Gazette 1993/21)**

(54) **APPARATUS FOR ULTRASONIC THERAPEUTIC TREATMENT**

VORRICHTUNG ZUR THERAPIE MITTELS ULTRASCHALL

APPAREIL DE TRAITEMENT THERAPEUTIQUE PAR ULTRASONS

(84) Designated Contracting States:
**AT BE CH DE DK ES FR GR IE IT LI NL PT SE**

(30) Priority: **25.02.1992 GB 9204021**

(43) Date of publication of application:
**14.12.1994 Bulletin 1994/50**

(73) Proprietors:
• **YOUNG, Michael John Radley**
**Ashburton, South Devon TQ13 7JX (GB)**
• **BRADNOCK, Brian R. D. P.**
**Edinburgh EH9 2DT (GB)**

(72) Inventors:
• **YOUNG, Michael John Radley**
**Ashburton, South Devon TQ13 7JX (GB)**
• **BRADNOCK, Brian R. D. P.**
**Edinburgh EH9 2DT (GB)**

(74) Representative:
**Gregory, Timothy Mark**
**T.M. Gregory & Co.,**
**26 Cyril Street**
**Northampton NN1 5EL (GB)**

(56) References cited:
**EP-A- 0 324 711          AT-A-  388 864**
**US-A- 3 847 141          US-A- 4 989 613**
**US-A- 5 143 069**

## Description

The present invention relates to an ultrasonic therapeutic treatment device.

The application of ultrasound in diagnostic scanning techniques and therapeutic treatment of specific medical conditions has been widely reported in technical literature over the last 20-30 years. However, we have found no relevant reference to the use of frequencies in the range 30-100 kHz. The reported applications of therapeutic ultrasound relate almost exclusively to use of frequencies in the MHz band. One example of this is shown in US-A-3847141 which discloses an ultrasonic bone densitometer using a frequency in the MHz band. Also EP-A-324711 discloses use of energy at 1.5 MHz for healing bone fractures. US-A-4989613 on which the preamble of Claim 1 is based discloses another apparatus for confirming suspected bone fractures using intrasonic energy at a frequency below 20 kHz.

This use of MHz frequencies stems from the concept that therapeutic treatment using ultrasonic energy should be directed accurately to a well defined region of tissue and that this is best achieved with a finely focused beam which in turn dictates the use of high frequencies. Similar arguments apply to the selection of frequencies for ultrasonic imaging applications. For example the characteristic wavelength corresponding to a 3 MHz transmission through soft tissue is about 0.5mm; but at 40 kHz the wavelength would be approximately 37.5mm.

Furthermore, it is known that the attenuation of ultrasonic waves increases with increasing frequency. The general effect of high frequency transmission is to produce relatively high energy absorption rates close to the entry surface and for the effect to fall off with increasing depth. It might therefore be concluded that for a given power input there is a greater likelihood of potentially harmful side effects in tissue near the surface than for a corresponding low frequency input. When treating deep tissue injuries this consideration becomes very important since in order to transmit enough energy to the required region, the risk of excessive absorption in the surface layers may become unreasonably high when applying therapeutic ultrasound in the MHz band. For this reason energy levels are limited by the requirement that power input should not exceed 3 watts/cm$^2$.

By selecting an operating frequency band in the range 30-130 kHz a good wave penetration through deep muscle tissue is ensured and frequencies which are known to result in high attenuation in bone tissue are avoided.

Experience of this form of therapy reveals a need to effect treatment in regions of the body which naturally inhibit access, particularly the hands and feet. In such cases the use of a specially shaped therapy head may greatly facilitate the treatment of an injured joint. It is recognised that under such circumstances repetitive cyclic movement of the treatment head over the skin surface may be difficult and that the need to avoid the establishment of standing waves must therefore be satisfied by an alterative technique.

It is an object of the present invention to address the problem of probe access whilst at the same time choosing an operating frequency which optimises the relationship between wave penetration, treatment intensity and minimum risk of tissue damage.

According to one aspect of the present invention there is provided an apparatus to treat muscular injuries below a body surface or to diagnose bone fractures which comprises piezo electric means to generate ultrasonic energy and head means adapted to be applied closely to the body surface, characterised in that the ultrasonic energy is generated at a frequency in the range 20-120 kHz, and in that means are provided to transfer said ultrasonic energy to the head means and thereby into the body.

The frequency range employed permits significantly higher energy dosage to deep seated injuries without causing damage to surface tissue as might be the case using more conventional high frequency radiation.

Methods utilising the apparatus of the invention by which low frequency vibrations are introduced through a moulded plastic head permit effective coupling to irregularly shaped surfaces and ensure even energy distribution throughout the targeted volume of tissue.

The head may be machined or moulded from a range of dense polymers including acetal, polypropylene and polycarbonate. These and similar materials all permit the transmission of low amplitude ultrasound in the frequency range 30-100 kHz with very low energy absorbtion. The head is machined from plastics material which is chosen because its specific impedance closely matches that of human soft tissue. As an example, acetal may be used, in which case figures are: W acetal = 1.86 x 10$^6$ kg m-$^2$sec-$^1$; W soft tissue = 1.65 x 10$^6$ kg m-$^2$sec-$^1$. This allows good coupling using virtually any fluid which excludes air from the head/tissue interface. It must be noted that this approach could not be used for radiation in the MHz band since absorption in the head material would be very high at these frequencies.

Clinical trials using this technique have so far proved that the treatment is effective in rapidly reducing pain levels related to conditions such as: ankle sprains, anterior knee pain, lower back pain, neck and wrist sprains, and muscle spasm such as may be related to spasticity.

The apparatus of the invention may be used to provide a method for the application of low frequency vibrations to all injured limb in order to provide a simple screening test for suspected fractures.

The presence of a hairline fracture would normally be detected by radiography but if a high proportion of X-ray examinations prove negative there is a strong argument for conducting a simple preliminary examination to identify those cases presenting definable symptoms. When ultrasound is transmitted though bone tissue containing a fine crack the wave is partially reflected at the interface between the two sections of bone, due to the mechanical discontinuity in the transmission medium. Some energy is however absorbed at the site of the injury causing a local transient sensation of pain which provides an initial indication of a fracture. It should be emphasised that this preliminary indication may result from a routine therapeutic treatment of an injury and not a specific intention to test for a fracture.

Embodiments of the invention will now be described more particularly, by way of example, and with reference to the accompanying drawings, in which:

Figure 1 is a graphical representation of the velocity and stress distributed across the transducer and head indicating the travelling wave amplitude in the head;

Figure 2 shows a schematic view of a piezo electric transducer and head assembly;

Figures 3 to 7 show alternative head members for use in an apparatus embodying the present invention;

Figure 8 shows the electrical drive system for said transducer and head;

Figure 9 shows an apparatus embodying the invention and which incorporates an accelerometer; and

Figures 10 and 11 show an apparatus adapted for use on an injured hand.

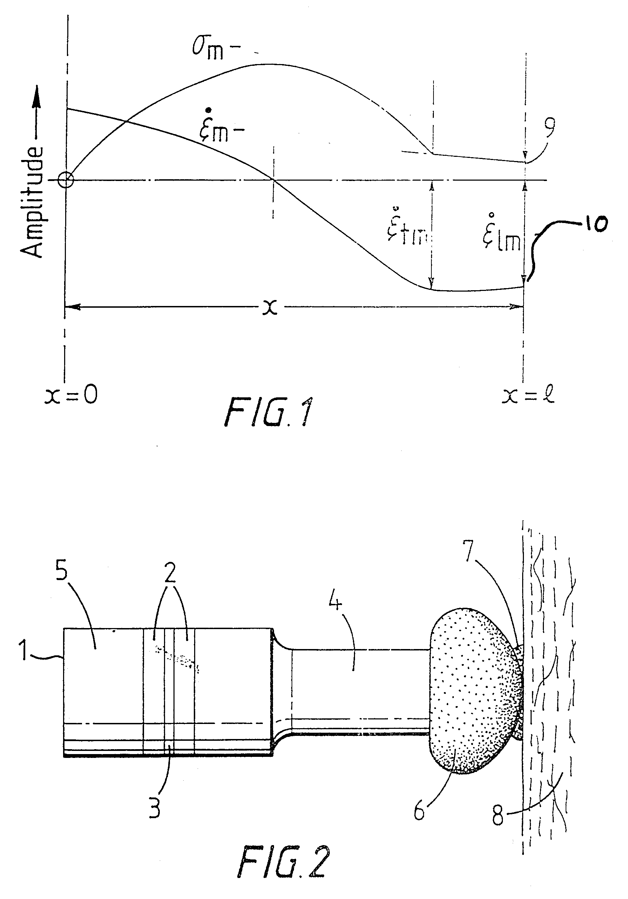

Referring now to the drawings; Figure 2 shows a vibrator in the form of a PZT sandwich transducer incorporating a backplate 5, PZT ceramic rings 2 (piezo electric transducer means), electrode 3 and stepped output section 4. This vibrator transmits waves at a predetermined frequency through a shaped plastic head 6 into tissue 8 via coupling medium 7.

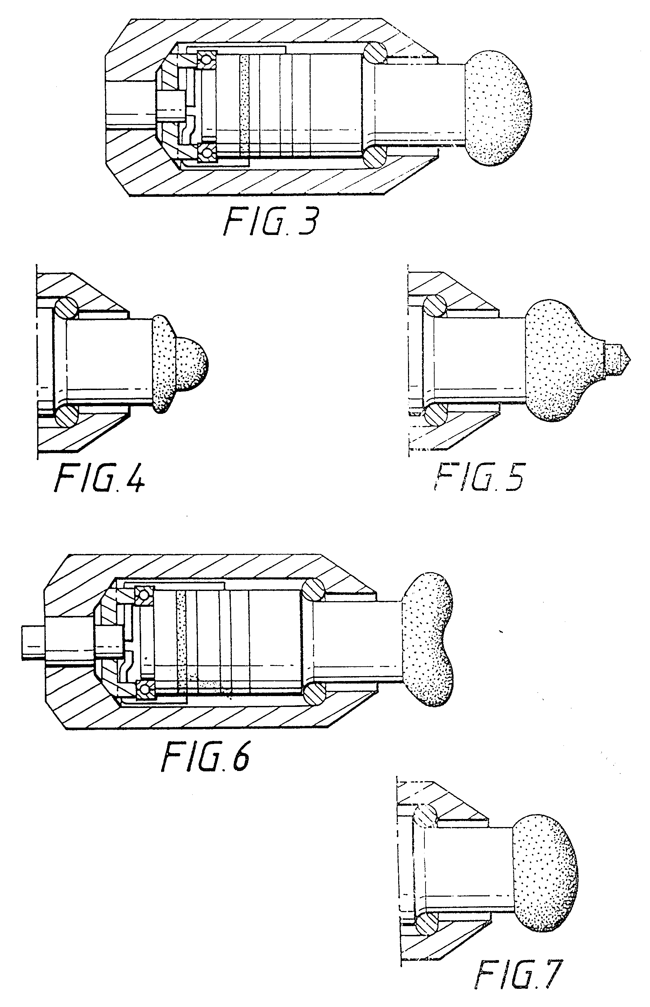

Figure 1 shows the waveform in the system. A standing wave is established in the transducer with output velocity amplitude $\mathring{\xi}_m$, and this is transmitted through the shaped therapy head 6 emerging as a travelling wave of amplitude $\mathring{\xi}_{LM}$. The velocity $\mathring{\xi}_m$, 10 and pressure or stress $\sigma_m$, 9 wave amplitudes in the plastics head are seen to be relatively constant under loaded conditions. They therefore represent the travelling wave amplitude for energy transmitted into the patient. This condition is establislied due to reflection at the transducer/head interface and almost complete transmission at the head/tissue interface. The shape of the head may be varied at least according to the examples given in Figures 3 to 7. This characteristic reflects the particular properties of the plastics chosen for the head construction which allow accurate control of frequency and amplitude. For given transducer dimensions the shape and size of the head can be varied between wide limits whilst maintaining a controlled output power.

In operation the energy transmitted to the subject tissue must not result in standing waves since this might cause excessive local absorption. This would normally be avoided by moving the head over the tissue surface during treatment; however when treating the hand or other inaccessible area such movement may be inhibited by the head shape and an alternative method must be used. The broad band transmission characteristic of the head permits the use of frequency modulation derived from the system shown in the drawings.

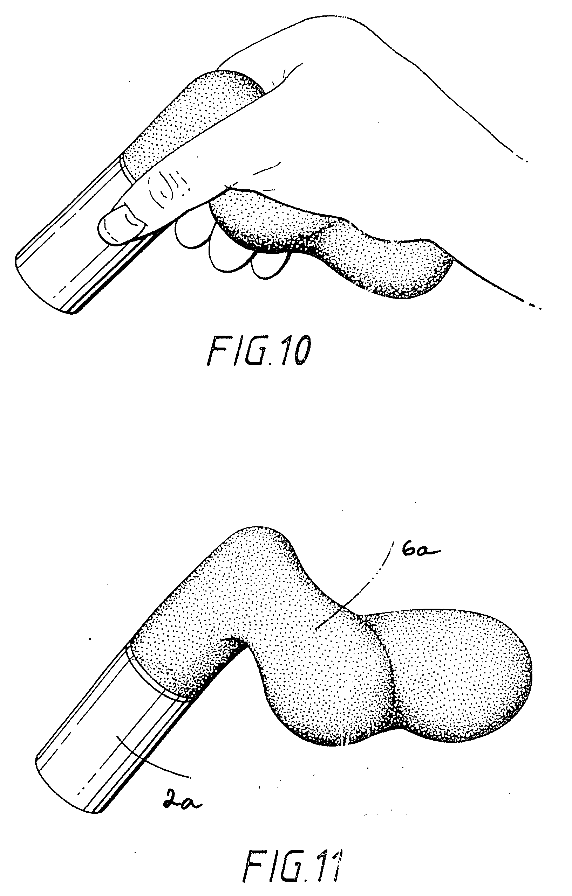

A further advantage which derives from the use of a plastics, mouldable head is the ability to employ a shaped head designed to give maximum contact in locations with difficult access eg hand and feet. For example in sever cases of rheumatoid arthritis the head could be moulded to form a hand grip which when held by the patient would permit general treatment of the hand joints simultaneously. This is shown in Figures 10 and 11. When the power input is supplied by battery means or some other transportable source, it and the transducer 2a may be located within the stem of a walking stick or cane, the grip 6a of which comprises the head 6.

This invention offers an improved method and means for the therapeutic treatment of deep seated soft tissue injuries by ensuring that adequate power is safely transmitted to the affected region. It offers a novel means of treating irregularly shaped areas using moulded or machined heads that allow good transmission of energy without the need to traverse the surface.

According to the invention, there is provided an apparatus which offers a major benefit in the technique available to monitor the treatment power delivered to a patient.

It is known that the intensity of transmitted ultrasound, I, is related to displacement amplitude, $\xi$, by the expression; $I = \frac{1}{2}\rho c\omega^2 \xi_o^2$ , where $\rho$ is the head material density, c the phase velocity and $\omega$, the angular frequency defined as $2\pi f$.

Since $\rho c$ is the wave impedance of the head material which by design matches that of the treated tissue, then $I = \xi^2$. If we monitor the displacement amplitude within the treatment head it is a relatively simple matter to obtain a linear signal, using a differentiating amplifier. This method offers much great reliability than the current technique which depends on monitoring the electrical signal to the transducer. Any variation in the transducer performance would therefore cause a power measuring error.

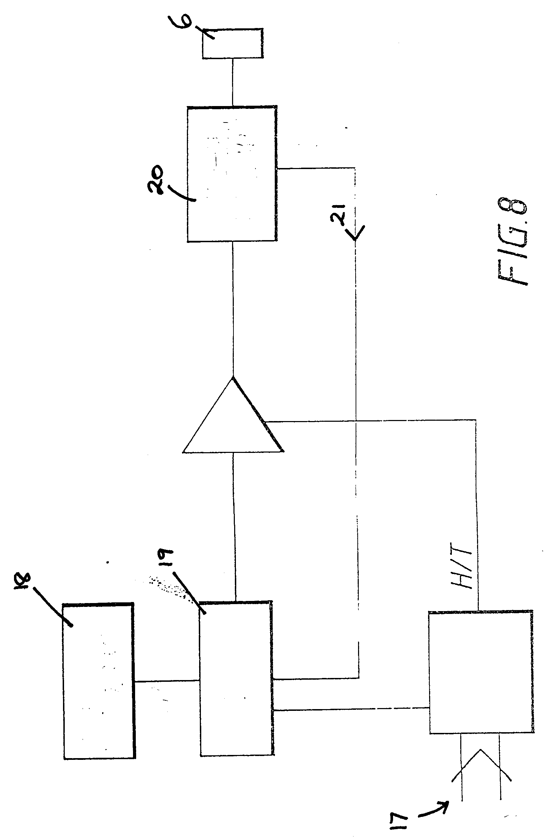

Figure 8 shows the electrical drive system comprising a 50 Hz supply 17 and VCO modulation unit 18 output to phase locked loop 19, which is also controlled by feedback signal 21 from matching network 20. The output of the matching network 20 controls operation of the head 6.

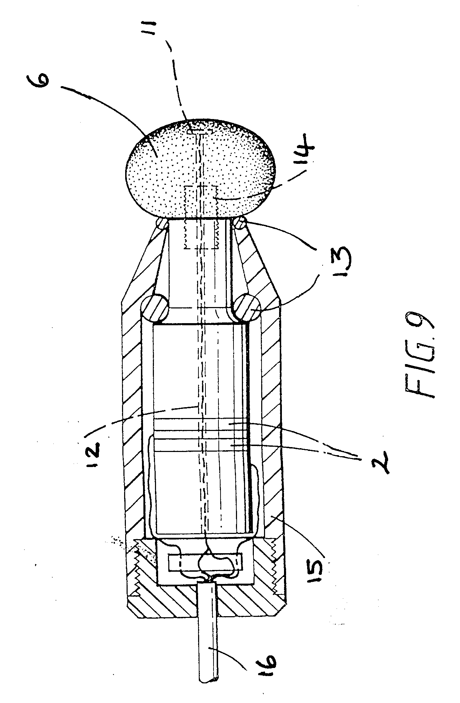

Figure 9 shows a head 6 with a displacement sensor or accelerometer incorporated therein. This enables the displacement amplitude or vibrations transmitted to the patient to be determined. The measurements may be transmitted to indicating means for the user to control the power input. The output of the displacement sensor may alternatively be used to control directly the power input. In other respects the apparatus of Figure 9 is conventional, having the treatment head 6 and accelerometer 11 attached by means of threaded connector 14, and via seals 13, to the main body of the apparatus and the piezo electric transducer 2. An axial hole 12 extending longitudinally of the apparatus allows connection of the accelerometer 11 to connection cable 16. The transducer assembly is housed within a plastics casing 15.

## Claims

1. An apparatus to treat muscular injuries below a body surface or to diagnose bone fractures, comprising piezo-electric means (2) to generate ultrasonic energy and head means (6) adapted to be applied closely to the body surface, characterised in that said ultrasonic energy is generated at a frequency in the range 20-120 kHz and in that means are provided to transfer said ultrasonic energy to the head means and thereby into the body.

2. An apparatus as claimed in Claim 1, characterised in that the frequency is in the range of 30-100 kHz.

3. An apparatus as claimed in either Claim 1 or Claim 2, characterised in that the frequency is in the region of 40 kHz.

4. An apparatus as claimed in any one of the preceding claims, characterised in that the material of the head means (6) is adapted to transmit ultrasonic vibrations at frequencies in the region of 20-120 kHz and is adapted to be shaped appropriately for the treatment being given.

5. An apparatus as claimed in Claim 4, characterised in that said head means (6) comprises a shaped plastics material, such as acetal, polypropylene or polycarbonate.

6. An apparatus as claimed in Claim 4 or Claim 5, characterised in that means are provided in said head means (6) to determine displacement amplitude at a treatment surface of the head.

7. An apparatus as claimed in any one of Claims 4 to 6, characterised in that said head means (6) is shaped to be manually grippable and to be accommodated within a hand.

8. An apparatus as claimed in Claim 7, characterised in that the head means forms a hand grip of a cane or walking stick, and in that the piezo electric means are accommodated within a stem of the cane.

## Patentansprüche

1. Vorrichtung zum Behandeln von Muskelverletzungen unter einer Körperoberfläche oder zum Diagnostizieren von Knochenfrakturen, umfassend piezoelektrische Mittel (2) zum Erzeugen von Ultraschallenergie und eine Kopfeinheit (6), die fest an der Körperfläche ansetzbar ist, dadurch gekennzeichnet, daß die Ultraschallenergie mit einer Frequenz im Bereich von 20 - 120 kHz erzeugt wird und daß Mittel zum Übertragen der Ultraschallenergie zur Kopfeinheit und dadurch in den Körper vorgesehen sind.

2. Vorrichtung nach Anspruch 1, dadurch gekennzeichnet, daß die Frequenz im Bereich von 30 - 100 kHz liegt.

3. Vorrichtung nach Anspruch 1 oder 2, dadurch gekennzeichnet, daß die Frequenz im Bereich von 40 kHz liegt.

4. Vorrichtung nach einem der vorangehenden Ansprüche, dadurch gekennzeichnet, daß der Werkstoff der Kopfeinheit (6) Ultraschallschwingungen auf Frequenzen im Bereich von 20 - 120 kHz zu übertragen vermag und für die gegebene Behandlung zweckmäßig formbar ist.

5. Vorrichtung nach Anspruch 4, dadurch gekennzeichnet, daß die Kopfeinheit 6 aus einem geformten Kunststoff, wie Acetal, Polypropylen oder Polycarbonat, besteht.

6. Vorrichtung nach Anspruch 4 oder 5, dadurch gekennzeichnet, daß in der Kopfeinheit (6) Mittel zum Bestimmen einer Wegamplitude an einer Behandlungsfläche des Kopfes vorgesehen sind.

7. Vorrichtung nach einem der Ansprüche 4 bis 6, dadurch gekennzeichnet, daß die Kopfeinheit (6) so geformt ist, daß sie von Hand ergreifbar und in einer Hand unterbringbar ist.

8. Vorrichtung nach Anspruch 7, dadurch gekennzeichnet, daß die Kopfeinheit einen Handgriff eines Geh- oder Spazierstocks bildet und daß die piezoelektrischen Mittel in einem Schaft des Geh- oder Spazierstocks untergebracht sind.

## Revendications

1. Appareil pour traiter les blessures musculaires sous la surface du corps ou pour diagnostiquer des fractures osseuses, comprenant un moyen piézoélectrique (2) pour générer de l'énergie ultrasonique et un moyen de tête (6) adapté pour être appliqué à proximité de la surface du corps, caractérisé en ce que ladite énergie ultrasonique est générée à une fréquence dans la plage de 20-120 kHz et en ce que des moyens sont prévus pour transférer ladite énergie ultrasonique au moyen de tête et par ce biais dans le corps.

2. Appareil selon la revendication 1, caractérisé en ce que la fréquence est dans la plage de 30-100 kHz.

3. Appareil selon l'une ou l'autre des revendications 1 et 2, caractérisé en ce que la fréquence est de l'ordre de 40 kHz.

4. Appareil selon l'une quelconque des revendications précédentes, caractérisé en ce que le matériau du moyen de tête (6) est adapté pour transmettre des vibrations ultrasoniques à des fréquences de l'ordre de 20-120 kHz et est adapté pour avoir une forme appropriée pour le traitement donné.

5. Appareil selon la revendication 4, caractérisé en ce que ledit moyen de tête (6) comprend un matériau plastique mis en forme, tel qu'un acétal, un polypropylène ou un polycarbonate.

6. Appareil selon la revendication 4 ou la revendication 5, caractérisé en ce que des moyens sont prévus dans ledit moyen de tête (6) pour déterminer l'amplitude de déplacement au niveau d'une surface de traitement de la tête.

7. Appareil selon l'une quelconque des revendications 4 à 6, caractérisé en ce que ledit moyen de tête (6) est mis en forme de manière à pouvoir être saisi manuellement et à être reçu dans la main.

8. Appareil selon la revendication 7, caractérisé en ce que le moyen de tête forme un pommeau d'une canne ou d'un bâton de marche, et en ce que le moyen piézoélectrique est reçu à l'intérieur d'une tige de la canne.

FIG.1

FIG.2

FIG. 3

FIG. 4

FIG. 5

FIG. 6

FIG. 7

FIG.8

FIG.9

FIG.10

FIG.11